# EUROPEAN PATENT SPECIFICATION

(11) **EP 3 864 005 B1**
(45) Date of publication and mention of the grant of the patent: **07.12.2022**
(21) Application number: 19783346.0
(22) Date of filing: 11.10.2019
(51) Int. Cl.: C07C 407/00, C08K 5/14, C08K 3/01, B65D 85/84, C08J 3/22

(54) **SOLID ORGANIC PEROXIDE COMPOSITION**
FESTE ORGANISCHE PEROXIDZUSAMMENSETZUNG
PRÉPARATION DE PEROXYDE ORGANIQUE SOLIDE

(30) Priority: 12.10.2018 EP 18200136
(43) Date of publication of application: 18.08.2021
(73) Proprietor: Nouryon Chemicals International B.V., 6824 BM Arnhem (NL)
(72) Inventor: FRIJLINK, Wilhelm, Klaas, 8017 BZ Zwolle (NL); WAANDERS, Petrus, Paulus, 7471 SL Goor (NL); NUYSINK, Johan, 7462 EV Rijssen (NL); KOERS, Rudy, 7418 AM Deventer (NL)
(74) Representative: LKGlobal UK Ltd.
(86) International application number: PCT/EP2019/077671
(87) International publication number: WO 2020/074740

(56) References cited:
- EP-A1- 3 034 551
- US-A1- 2008 221 269
- US-A1- 2011 118 400
- US-A1- 2018 051 160
- US-A1- 2018 244 883

## Description

The invention relates to a solid organic peroxide composition that can be suitably stored and transported in stainless steel containers.

Liquid organic peroxide compositions, generally containing organic peroxide diluted with an organic solvent or suspended or emulsified in water, are conventionally stored and transported in HDPE-based containers or stainless steel containers. Corrosion of the so-filled stainless steel containers is not an issue.

Solid organic peroxides are conventionally stored and transported in HDPE-based containers or LDPE-based bags. However, handling of small LDPE bags is labor intensive and may cause dusting, with consequential safety issues, whereas transportation of solid peroxydicarbonates and diacyl peroxides in big bags (super sacks or flexible IBCs) is not allowed by UN regulations. Furthermore, safety requirements on industrial plants and factories may require a container to have an electrical resistivity that can only be obtained with stainless steel containers. In addition, the durability of HDPE containers is limited to about 5 years.

In storing and transporting certain solid organic peroxides in stainless steel containers, it has turned out that the containers tend to corrode. This corrosion not only limits the lifetime of the container, but also releases iron ions (and potentially other metal ions) in the peroxide. Transition metals, such as iron, are known to catalyse peroxide decomposition, which is evidently undesired.

It is therefore an object of the present invention to provide a solid organic peroxide composition that can be suitably stored and transported in stainless steel containers, without corrosion of the container.

Investigations towards the cause of the corrosion have resulted in the conclusion that it is the residual chloroformate or acid chloride in the organic peroxide which, in combination with moisture or residual water in the product, leads to the formation of HCI fumes. These HCI fumes lead to corrosion of the stainless steel container.

Solid organic peroxides that are prepared from chloroformates are peroxydicarbonates. Peroxydicarbonates are conventionally prepared by reacting a chloroformate with hydrogen peroxide in alkaline medium.

Solid organic peroxides that are prepared from acid chlorides are diacyl peroxides. Diacyl peroxides are conventionally prepared by reacting an acid chloride with hydrogen peroxide in alkaline medium.

Upon transport and storage of these solid organic peroxides in stainless steel containers, the HCI fumes tend to corrode the stainless steel in the presence of oxygen and moisture.

It has now been found that solid peroxydicarbonate and diacylperoxide compositions that contain a small amount of an HCI scavenger can be safely stored and transported in stainless steel containers without leading to corrosion.

At the same time, these compositions are safe and stable, meaning that the HCI scavenger does not promote the decomposition of the organic peroxide, can be mixed with the organic peroxide without significant segregation, and remains flowable.

The present invention therefore relates to an organic peroxide composition comprising:
- at least 40 wt% - based on the weight of the entire composition - of an organic peroxide that is solid at room temperature, said organic peroxide being selected from peroxydicarbonates and diacylperoxides,
- 0.001-5 wt% - based on the weight of organic peroxide in the composition - of an HCI scavenger that is solid at room temperature.

The invention also relates to a packaged organic peroxide composition comprising said organic peroxide composition in a stainless steel container.

Suitable solid HCI scavengers include
- metal carboxylates, which include the metal salts of mono-, di- and tri-carboxylic acids. Examples of metal salts of mono-carboxylic acids are metal stearates, metal lactates, and metal lactylates, such as Ca, Mg, Zn, Al, Na, K, and Li stearates, lactates, or lactylates; more preferably calcium stearate and calcium stearoyl-2-lactylate. Examples of metal salts of dicarboxylic acids are metal hexahydrophthalates or metal bicyclic[2.2.1]heptane dicarboxylates, such as Ca, Mg, Zn, Al, Na, K, and Li hexahydrophthalates or bicyclic[2.2.1]heptane dicarboxylates. Examples of metal salts of tricarboxylic acids are metal citrates, such as Ca, Mg, Zn, Al, Na, K, and Li mono-, di-, and tricitrates, more in particular sodium monocitrate or potassium monocitrate.
- metal carbonates and metal bicarbonates, such as Ca, Mg, Zn, Na, K, or Li carbonate, or Na, K, or Li bicarbonate. A preferred (bi)carbonate is calcium carbonate;
- metal oxides, such as CaO, MgO, and ZnO, with CaO being the preferred metal oxide;
- metal silicates, such as calcium silicate or magnesium silicate
- anionic clays;
- and combinations thereof.

Anionic clays - also called hydrotalcites or layered double hydroxides - have a crystal structure consisting of positively charged layers built up of specific combinations of divalent and trivalent metal hydroxides between which there are anions and water molecules. Hydrotalcite is an example of a naturally occurring anionic clay, in which carbonate is the predominant anion and the layers contain Mg and Al. Various natural, synthetic, and modified forms with other di- and/or trivalent metals and/or anions (including nitrate anions, organic anions and pillaring anions) are known. The anionic clay should be able to exchange the anions in its interlayer for chloride anions. Preferred anionic clays are anionic clays with carbonate or hydroxide anions in the interlayers.

The metal carboxylates, metal oxides, metal silicates, and metal (bi)carbonates neutralize the HCI. The anionic clays exchange anions (e.g. carbonate) for chloride ions, thereby encapsulating the chloride ions in the clay structure.

Suitable solid peroxydicarbonates are dicetyl peroxydicarbonate, dimyristyl peroxydicarbonate, dicyclohexyl peroxydicarbonate, and di(tert-butylcyclohexyl) peroxydicarbonate.

Suitable solid diacylperoxides include aliphatic and aromatic diacylperoxides. Preferred aliphatic diacylperoxides are those with aliphatic chains of at least 10 carbon atoms, such as dilauroyl peroxide.

Preferred aromatic diacylperoxides include dibenzoyl peroxide and substituted dibenzoyl peroxides, such as di(2-methylbenzoyl)peroxide, di(4-methylbenzoyl)peroxide, and di(2,4-dichlorobenzoyl)peroxide.

Peroxydicarbonates and diacylperoxides find use in processes for enhancing the melt strength of polypropylene (towards high melt strength polypropylene; HMS-PP) by extruding said polypropylene in the presence of the peroxydicarbonate or diacylperoxide. The present invention therefore also relates to the use of the composition according to the present invention for the modification of polypropylene, such as the production of HMS-PP.

Acid scavengers like calcium stearate, calcium stearoyl-2-lactylate, calcium lactate, and/or anionic clays are already used as additives in a polypropylene (modification) process for the purpose of trapping acidic catalyst residues that may deteriorate the polypropylene. This means that the introduction of calcium stearate, calcium stearoyl-2-lactylate, calcium lactate, and/or anionic clays, but also calcium carbonate (the carbonate is released as CO₂ during polypropylene modification) via the composition according to the present invention, does not introduce any new materials in the modified polypropylene. Therefore, calcium stearate, calcium stearoyl-2-lactylate, calcium lactate, anionic clays, and/or calcium carbonate are preferred HCI scavengers in the composition of the present invention.

Peroxydicarbonates specifically suitable for this polypropylene modification are dicetyl peroxydicarbonate, dimyristyl peroxydicarbonate, and di(tert-butylcyclohexyl) peroxydicarbonate. A diacyl peroxides specifically suitable for this polypropylene modification is di(4-methylbenzoyl)peroxide. Therefore, in a preferred embodiment, the composition according to the present invention contains one or more of these peroxydicarbonates and diacyl peroxides.

Even more preferred are compositions containing a peroxydicarbonate or diacyl peroxide selected from the group consisting of dicetyl peroxydicarbonate, dimyristyl peroxydicarbonate, di(tert-butylcyclohexyl) peroxydicarbonate, and di(4-methylbenzoyl)peroxide in combination with an HCI scavenger selected from the group consisting of calcium stearate, calcium stearoyl-2-lactylate, anionic clay, and/or calcium carbonate.

The most preferred organic peroxides are dicetyl peroxydicarbonate and di(4-methylbenzoyl)peroxide.

The organic peroxide composition according to the present invention comprises at least 40 wt%, preferably at least 50 wt%, more preferably at least 60 wt%, even more preferably at least 85 wt%, and most preferably at least 90 wt% - based on the weight of the entire composition - of an organic peroxide that is solid at room temperature, said organic peroxide being selected from peroxydicarbonates and diacylperoxides.

The organic peroxide composition according to the present invention comprises 0.001-5 wt%, preferably 0.005-2.5 wt%, and most preferably 0.01-0.5 wt% - based on the weight of organic peroxide in the composition - of an HCI scavenger that is solid at room temperature.

Other components that can be present in the organic peroxide composition are water, phlegmatizers or diluents, or additives, provided that the organic peroxide composition remains a solid material.

The composition according to the present invention can have the form of a physical mixture of organic peroxide particles and HCI scavenger particles.

Alternatively, it has the form of particles (powder, flakes, granules, pills, pellets, or other solid particle form) containing both the peroxydicarbonate and HCI scavenger in one particle.

The organic peroxide composition according to the present invention can be prepared in several ways.

In one embodiment, the organic peroxide and the HCI scavenger are physically mixed. This mixing can be performed before addition of the composition to the stainless steel container for storage and transport. Alternatively, the organic peroxide and the HCI scavenger can be added to the stainless steel container individually, after which the ingredients are mixed inside the container.

Suitable mixing equipment includes tumble mixers and rotating screw mixers (e.g. Nauta mixers). Alternatively, the solids can be dosed by in-line mixing, e.g. by dosing to a rotating screw mixer or a conveying screw, under continuous mixing.

In a second embodiment, the HCI scavenger is added to the reactor in which the organic peroxide is produced. According to this embodiment, the HCI scavenger can be present during the reaction of the chloroformate or acid chloride with hydrogen peroxide in alkaline medium, or can be added to the reactor after the reaction has been completed.

In a third embodiment, the HCI scavenger is added to molten organic peroxide. The resulting product can then be transformed into solid particles by, e.g. extrusion or granulation.

The present invention also relates to a packaged organic peroxide composition comprising the organic peroxide composition as described above in a stainless steel container.

The present invention also relates to a stainless steel container comprising:
- a solid composition comprising at least 40 wt%, preferably at least 50 wt%, more preferably at least 60 wt%, even more preferably at least 85 wt%, and most preferably at least 90 wt% - based on the weight of the entire solid composition - of an organic peroxide that is solid at room temperature and is selected from peroxydicarbonates and diacylperoxides,
- an HCI scavenger that is solid at room temperature, in an amount of at least 0.001 wt%, preferably 0.001-5 wt%, more preferably 0.005-2.5 wt%, and most preferably 0.01-0.5 wt% - based on the weight of organic peroxide present in the container. According to one embodiment, the HCI scavenger and the solid composition can be admixed according to one of the preparation embodiments described above in order to form an organic peroxide composition according to the present invention. In another embodiment, the HCI scavenger is not admixed with the solid composition, but is present in a separate compartment within the container. Said separate compartment should be permeable to HCI fumes.

As explained above, the filled stainless steel container will not be subject to corrosion by its contents. The term "container" refers to any type of packaging that can be closed and can be used to store and transport solid organic peroxide compositions.

The container preferably has a size of 200 to 4000 liters, more preferably 500-1500 liters. This includes stainless steel intermediate bulk containers (IBC's). A specifically preferred type of container is a Cone Valve IBC, which is specifically suited for powders as it prevents typical powder flow problems such as bridging, blockages, segregation, flushing, and core-flow. Such containers are available from Matcon^{®}.

As mentioned above, the organic peroxide composition according to the present invention finds use in the modification of polypropylene, in particular the production of high melt strength polypropylene (HMS-PP). In this modification, the composition is added to the polypropylene prior to or during extrusion. The composition may be added to the polypropylene after suspending it in water, dispersing it in an inert solvent such as isododecane, or in any solid physical form, e.g. flakes or powder. The quantity of organic peroxide to be used will depend on the desired degree of modification and on the type of polypropylene employed. Preferably, use is made of organic peroxide concentrations in the range of 0.1 to 3.0 g of peroxide per 100 g polypropylene, more preferably in the range of 0.5 to 2.0 g per 100 g polypropylene; all calculated as pure and dry organic peroxide.

### EXAMPLES

### Example 1

A composition was prepared by tumble mixing a HDPE flask containing dicetyl peroxydicarbonate flakes (containing 97.5 wt% peroxide and 0.3 wt% cetylchloroformate) with different amounts (in wt% based on the organic peroxide weight) of different acid scavengers.

Experiments with an additional amount of cetylchloroformate (0.9 wt%) were also performed.

The following acid scavengers were used: calcium stearate, hydrotalcite (DHT-4A, ex-Kisuma), calcium carbonate.

Pre-cleaned smooth stainless steel (SS) 316L coupons (20x10x1 mm) were weighed and subsequently placed in a glass vial with approximately 1 g organic peroxide composition.

The open glass vial was placed into a 250 ml HDPE flask containing approximately 20 g organic peroxide composition. The HDPE flask was closed with a screw-cap and was stored for at least 12 days at 20°C.

After the storage period, the coupons were visually inspected for corrosion and, after the below cleaning procedure, re-weighed to determine the weight loss. According to the cleaning procedure, any rust was removed from the corroded coupons with a wetted sponge (water/abrasive creme). The coupons were rinsed with distilled water and then with acetone, dried in an oven at 60°C for 1 hour, and cooled to room temperature.

**Table 1**

| **Exp.** | **HCl scavenger** | **Stainless steel type** | **days at 20°C** | **Corrosion?** | **Weight loss (%)** | **Observations** |
|---|---|---|---|---|---|---|
| | | | | | | |

| **No acid scavenger** | | | | | | |
|---|---|---|---|---|---|---|
| 1 | - | 316L | 12 | ++ | 0.108 | clear corrosion, brown droplets and deterioration of coupon |
| 2 | - | 254 SMO | 12 | + | 0.027 | Corrosion, but less than 316L |
| | | | | | | |

| **Ca-stearate** | | | | | | |
|---|---|---|---|---|---|---|
| 3 | 0.05% wt% Ca-stearate | 316L | 14 | -- | | no corrosion |
| 4 | 0.25% wt% Ca-stearate | 316L | 60 | -- | | no corrosion |
| | | | | | | |

| **DHT-4A** | | | | | | |
|---|---|---|---|---|---|---|
| 5 | 0.01 wt% DHT-4A | 316L | 19 | -- | | no corrosion |
| 6 | 0.025 wt% DHT-4A | 316L | 42 | -- | | no corrosion |
| 7 | 0.2 wt% DHT-4A | 316L | 19 | -- | | no corrosion |
| | | | | | | |

| **CaCO3** | | | | | | |
|---|---|---|---|---|---|---|
| 8 | 0.016 wt% CaCO3 | 316L | 19 | -- | | no corrosion |
| 9 | 0.04 wt% CaCO3 | 316L | 19 | -- | | no corrosion |
| 10 | 0.1 wt% CaCO3 | 316L | 19 | -- | | no corrosion |
| | | | | | | |

| **Dicetyl peroxydicarbonate flakes with additional 0.9 wt% cetylchloroformate** | | | | | | |
|---|---|---|---|---|---|---|
| 11 | - | 316L | 14 | ++ | 0.220 | clear corrosion |
| 12 | 2.5 wt% Ca stearate | 316L | 19 | -- | | no corrosion |
| | | | | | | |

| **Influence of temperature on corrosion; no HCl scavenger** | | | | | | |
|---|---|---|---|---|---|---|
| 13 | 10°C | 316L | 12 | ++ | n.d. | clear corrosion |
| 14 | 20°C | 316L | 12 | ++ | n.d. | clear corrosion |

The results show that the organic peroxide composition, without HCI scavenger, corrodes 316L stainless steel. The results also show that it corrodes a higher quality stainless steel 254 SMO; a stainless steel that is resistant to dilute HCI solutions.

### Example 2

Several compositions prepared in Example 1 were tested for their corroding effects using the following test set-up:
Pre-cleaned smooth 316L stainless steel coupons (20x10x1 mm) were weighed and subsequently placed in a 250 ml HDPE flask: directly on top, or below a 21 g sample of the dicetyl peroxydicarbonate-containing composition.

The HDPE flask was closed with a screw-cap and was stored for at least 11 days at 20°C.

After the test, the coupons were inspected, cleaned and weighed as explained in Example 1.

**Table 2**

| **Exp.** | **HCl scavenger** | **days at 20°C** | **Corrosion?** | **Weight loss (%)** | **Observations** |
|---|---|---|---|---|---|
| | | | | | |

| **Coupon below the composition** | | | | | |
|---|---|---|---|---|---|
| 15 | - | 13 | ++ | 0.097 | Clear corrosion |
| 16 | 0.05 wt% Ca stearate | 11 | -- | | No corrosion |
| 17 | 0.1 wt% Ca stearate | 11 | -- | | No corrosion |
| 18 | 0.5 wt% Ca stearate | 13 | -- | | No corrosion |
| | | | | | |

| **Coupon on top of the composition** | | | | | |
|---|---|---|---|---|---|
| 19 | - | 13 | ++ | 0.187 | Clear corrosion |
| 20 | 0.05 wt% Ca stearate | 11 | -- | | No corrosion |
| 21 | 0.1 wt% Ca stearate | 11 | -- | | No corrosion |

These results again confirm that the HCI scavenger is able to prevent corrosion of all inner walls of a stainless steel container.

## Claims

1. Organic peroxide composition that is solid at room temperature, said composition comprising:
- at least 40 wt% - based on the weight of the entire composition - of an organic peroxide that is solid at room temperature, said organic peroxide being selected from peroxydicarbonates and diacylperoxides,
- 0.001-5 wt% - based on the weight of organic peroxide in the composition - of an HCI scavenger that is solid at room temperature.

2. Organic peroxide composition according to claim 1 wherein the composition comprises 0.005-2.5 wt%, preferably 0.01-0.5 wt% - based on the weight of said organic peroxide in the composition - of said HCI scavenger.

3. Organic peroxide composition according to claim 1 wherein the composition comprises at least 85 wt%, preferably at least 90 wt% - based on the weight of the entire composition - of said organic peroxide.

4. Organic peroxide composition according to any one of the preceding claims wherein the organic peroxide is a peroxydicarbonate selected from the group consisting of dicetyl peroxydicarbonate, dimyristyl peroxydicarbonate, dicyclohexyl peroxydicarbonate, and di(tert-butylcyclohexyl) peroxydicarbonate.

5. Organic peroxide composition according to any one of claims 1-3 wherein the organic peroxide is a diacylperoxide selected from the group consisting of aliphatic diacylperoxides with aliphatic chains of at least 10 carbon atoms, dibenzoyl peroxide, and substituted dibenzoyl peroxides.

6. Organic peroxide composition according to any one of the preceding claims wherein the HCI scavenger is selected from the group consisting of metal carboxylates, metal oxides, metal (bi)carbonates, metal silicates, anionic clays, and combinations thereof.

7. Organic peroxide composition according to claim 6 wherein the HCI scavenger is selected from the group consisting of calcium stearate, calcium stearoyl-2-lactylate, CaO, calcium carbonate, and anionic clays, preferably selected from the group consisting of hydrotalcite, calcium stearate, and calcium carbonate.

8. Packaged organic peroxide composition comprising the organic peroxide composition according to any one of claims 1-7 in a stainless steel container.

9. Stainless steel container comprising the organic peroxide composition according to any one of claims 1-7.

10. Stainless steel container comprising:
- a solid composition comprising at least 40 wt% - based on the weight of said solid composition - of an organic peroxide that is solid at room temperature and is selected from peroxydicarbonates and diacylperoxides,
- an HCI scavenger that is solid at room temperature, in an amount of at least 0.001 wt%, based on the weight of organic peroxide that is present in the stainless steel container.

11. Method for storing and/or transporting an organic peroxide composition that is solid at room temperature in a stainless steel container, said method involving the addition to a stainless steel container of:
(i) a solid composition comprising at least 40 wt% - based on the weight of said solid composition - of an organic peroxide that is solid at room temperature and is selected from peroxydicarbonates and diacylperoxides, and
(ii) at least 0.001 wt%, based on the weight of organic peroxide that is present in the stainless steel container, of an HCI scavenger that is solid at room temperature.

12. Process for producing the organic peroxide composition according to any one of claims 1-7, comprising the step of physically mixing said organic peroxide and said HCI scavenger.

13. Process for producing the organic peroxide composition according to any one of claims 1-7, wherein said HCI scavenger is added to the reactor in which the organic peroxide is produced.

14. Process for producing the organic peroxide composition according to any one of claims 1-7, comprising the step of adding said HCI scavenger to molten organic peroxide.

15. Use of an organic peroxide composition according to any one of claims 1-7 for the modification of polypropylene, preferably a modification that results in increased melt strength of the polypropylene (HMS-PP).

## Patentansprüche

1. Organische Peroxidzusammensetzung, die bei Raumtemperatur fest ist, wobei die Zusammensetzung Folgendes umfasst:
- mindestens 40 Gew.-% - auf das Gewicht der gesamten Zusammensetzung bezogen - eines organischen Peroxids, das bei Raumtemperatur fest ist, wobei das organische Peroxid unter Peroxydicarbonaten und Diacylperoxiden ausgewählt ist,
- 0,001-5 Gew.-% - auf das Gewicht von organischem Peroxid in der Zusammensetzung bezogen - eines HCl-Fängers, der bei Raumtemperatur fest ist.

2. Organische Peroxidzusammensetzung nach Anspruch 1, wobei die Zusammensetzung 0,005-2,5 Gew.-%, bevorzugt 0,01-0,5 Gew.-% - auf das Gewicht des organischen Peroxids in der Zusammensetzung bezogen - des HCl-Fängers umfasst.

3. Organische Peroxidzusammensetzung nach Anspruch 1, wobei die Zusammensetzung mindestens 85 Gew.-%, bevorzugt mindestens 90 Gew.-% - auf das Gewicht der gesamten Zusammensetzung bezogen - des organischen Peroxids umfasst.

4. Organische Peroxidzusammensetzung nach einem der vorhergehenden Ansprüche, wobei das organische Peroxid ein Peroxydicarbonat ist ausgewählt aus der Gruppe bestehend aus Dicetylperoxydicarbonat, Dimyristylperoxydicarbonat, Dicyclohexylperoxydicarbonat und Di(tert-butylcyclohexyl)peroxydicarbonat.

5. Organische Peroxidzusammensetzung nach einem der Ansprüche 1-3, wobei das organische Peroxid ein Diacylperoxid ist ausgewählt aus der Gruppe bestehend aus aliphatischen Diacylperoxiden mit aliphatischen Ketten von mindestens 10 Kohlenstoffatomen, Dibenzoylperoxid und substituierten Dibenzoylperoxiden.

6. Organische Peroxidzusammensetzung nach einem der vorhergehenden Ansprüche, wobei der HCl-Fänger aus der Gruppe ausgewählt ist bestehend aus Metallcarboxylaten, Metalloxiden, Metall(bi)carbonaten, Metallsilicaten, anionischen Tonen und Kombinationen davon.

7. Organische Peroxidzusammensetzung nach Anspruch 6, wobei der HCl-Fänger aus der Gruppe ausgewählt ist bestehend aus Calciumstearat, Calciumstearoyl-2-lactylat, CaO, Calciumcarbonat und anionischen Tonen, bevorzugt aus der Gruppe ausgewählt ist bestehend aus Hydrotalcit, Calciumstearat und Calciumcarbonat.

8. Verpackte organische Peroxidzusammensetzung umfassend die organische Peroxidzusammensetzung nach einem der Ansprüche 1-7 in einem Edelstahlbehälter.

9. Edelstahlbehälter umfassend die organische Peroxidzusammensetzung einem der Ansprüche 1-7.

10. Edelstahlbehälter umfassend:
- eine feste Zusammensetzung umfassend mindestens 40 Gew.-% - auf das Gewicht der festen Zusammensetzung bezogen - eines organischen Peroxids, das bei Raumtemperatur fest und unter Peroxydicarbonaten und Diacylperoxiden ausgewählt ist,
- einen HCl-Fänger, der bei Raumtemperatur fest ist, in einer Menge von mindestens 0,001 Gew.-%, auf das Gewicht von organischem Peroxid, das in dem Edelstahlbehälter vorliegt, bezogen.

11. Verfahren zum Speichern und/oder Transportieren einer organischen Peroxidzusammensetzung, die bei Raumtemperatur fest ist, in einem Edelstahlbehälter, wobei das Verfahren das Eingeben in einen Edelstahlbehälter involviert von:
(i) einer festen Zusammensetzung, die mindestens 40 Gew.-% - auf das Gewicht der festen Zusammensetzung bezogen - eines organischen Peroxids umfasst, das bei Raumtemperatur fest und unter Peroxydicarbonaten und Diacylperoxiden ausgewählt ist,
(ii) mindestens 0,001 Gew.-%, auf das Gewicht von organischem Peroxid, das in dem Edelstahlbehälter vorliegt, bezogen, eines HCl-Fängers, der bei Raumtemperatur fest ist.

12. Verfahren für die Herstellung der organischen Peroxidzusammensetzung nach einem der Ansprüche 1-7, umfassend den Schritt des physischen Mischens des organischen Peroxids und des HCl-Fängers.

13. Verfahren für die Herstellung der organischen Peroxidzusammensetzung nach einem der Ansprüche 1-7, wobei der HCl-Fänger in den Reaktor, in dem das organische Peroxid hergestellt wird, eingegeben wird.

14. Verfahren für die Herstellung der organischen Peroxidzusammensetzung nach einem der Ansprüche 1-7 umfassend den Schritt des Zugebens des HCl-Fänger zu geschmolzenem organischem Peroxid.

15. Verwendung einer organischen Peroxidzusammensetzung nach einem der Ansprüche 1 1-7 für die Modifikation von Polypropylen, bevorzugt eine Modifikation, die zu erhöhter Schmelzfestigkeit des Polypropylens (HMS-PP) führt.

## Revendications

1. Composition de peroxyde organique qui est solide à température ambiante, ladite composition comprenant :
- au moins 40 % en poids, sur la base du poids de l'ensemble de la composition, d'un peroxyde organique qui est solide à température ambiante, ledit peroxyde organique étant sélectionné parmi les peroxydicarbonates et les peroxydes de diacyle,
- de 0,001 à 5 % en poids, sur la base du poids de peroxyde organique dans la composition, d'un piégeur d'HCl qui est solide à température ambiante.

2. Composition de peroxyde organique selon la revendication 1 dans laquelle la composition comprend de 0,005 à 2,5 % en poids, de préférence de 0,01 à 0,5 % en poids, sur la base du poids dudit peroxyde organique dans la composition, dudit piégeur d'HCl.

3. Composition de peroxyde organique selon la revendication 1 dans laquelle la composition comprend au moins 85 % en poids, de préférence au moins 90 % en poids, sur la base du poids de l'ensemble de la composition, dudit peroxyde organique.

4. Composition de peroxyde organique selon l'une quelconque des revendications précédentes dans laquelle le peroxyde organique est un peroxydicarbonate sélectionné dans le groupe constitué par le peroxydicarbonate de dicétyle, le peroxydicarbonate de dimyristyle, le peroxydicarbonate de dicyclohexyle et le peroxydicarbonate de di(tert-butylcyclohexyle).

5. Composition de peroxyde organique selon l'une quelconque des revendications 1 à 3 dans laquelle le peroxyde organique est un peroxyde de diacyle sélectionné dans le groupe constitué par les peroxydes de diacyle aliphatiques ayant des chaînes aliphatiques d'au moins 10 atomes de carbone, le peroxyde de dibenzoyle et les peroxydes de dibenzoyle substitués.

6. Composition de peroxyde organique selon l'une quelconque des revendications précédentes dans laquelle le piégeur d'HCl est sélectionné dans le groupe constitué par les carboxylates métalliques, les oxydes métalliques, les (bi)carbonates métalliques, les silicates métalliques, les argiles anioniques et des combinaisons de ceux-ci.

7. Composition de peroxyde organique selon la revendication 6 dans laquelle le piégeur d'HCl est sélectionné dans le groupe constitué par le stéarate de calcium, le stéaroyl-2-lactylate de calcium, le CaO, le carbonate de calcium et les argiles anioniques, de préférence sélectionnés dans le groupe constitué par l'hydrotalcite, le stéarate de calcium et le carbonate de calcium.

8. Composition de peroxyde organique conditionnée comprenant la composition de peroxyde organique selon l'une quelconque des revendications 1 à 7 dans un récipient en acier inoxydable.

9. Récipient en acier inoxydable comprenant la composition de peroxyde organique selon l'une quelconque des revendications 1 à 7.

10. Récipient en acier inoxydable comprenant :
- une composition solide comprenant au moins 40 % en poids, sur la base du poids de ladite composition solide, d'un peroxyde organique qui est solide à température ambiante et sélectionné parmi les peroxydicarbonates et les peroxydes de diacyle,
- un piégeur d'HCl qui est solide à température ambiante, en une quantité d'au moins 0,001 % en poids, sur la base du poids de peroxyde organique qui est présent dans le récipient en acier inoxydable.

11. Procédé de stockage et/ou de transport d'une composition de peroxyde organique qui est solide à température ambiante dans un récipient en acier inoxydable, ledit procédé impliquant l'ajout à un récipient en acier inoxydable de :
(i) une composition solide comprenant au moins 40 % en poids, sur la base du poids de ladite composition solide, d'un peroxyde organique qui est solide à température ambiante et est sélectionné parmi les peroxydicarbonates et les peroxydes de diacyle, et
(ii) au moins 0,001 % en poids, sur la base du poids de peroxyde organique qui est présent dans le récipient en acier inoxydable, d'un piégeur d'HCl qui est solide à température ambiante.

12. Procédé de production de la composition de peroxyde organique selon l'une quelconque des revendications 1 à 7, comprenant l'étape de mélange physique dudit peroxyde organique et dudit piégeur d'HCl.

13. Procédé de production de la composition de peroxyde organique selon l'une quelconque des revendications 1 à 7, dans lequel ledit piégeur d'HCl est ajouté au réacteur dans lequel le peroxyde organique est produit.

14. Procédé de production de la composition de peroxyde organique selon l'une quelconque des revendications 1 à 7, comprenant l'étape d'ajout dudit piégeur d'HCl au peroxyde organique fondu.

15. Utilisation d'une composition de peroxyde organique selon l'une quelconque des revendications 1 à 7 pour la modification de polypropylène, de préférence une modification qui entraîne une résistance accrue à l'état fondu du polypropylène (HMS-PP).
